# EUROPÄISCHE PATENTSCHRIFT

(11) **EP 0 819 409 B1**
(45) Veröffentlichungstag und Bekanntmachung des Hinweises auf die Patenterteilung: **09.04.2003**
(21) Anmeldenummer: 97111665.2
(22) Anmeldetag: 09.07.1997
(51) Int. Cl.: A61B 18/12

(54) **Verfahren zum Betrieb eines Hochfrequenz-Chirurgiegerätes und Hochfrequenz-Chirurgiegerät**
HF electrosurgical device and method of operation
Générateur électrochirurgical et son procédé de fonctionnement

(30) Priorität: 15.07.1996 DE 19628482
(43) Veröffentlichungstag der Anmeldung: 21.01.1998
(73) Patentinhaber: Berchtold Holding GmbH, 78532 Tuttlingen (DE)
(72) Erfinder: Schilling, Bertram, 78194 Mauenheim (DE); Tockweiler, Udo, 78194 Immendingen (DE); Hill, Wolfram, 79102 Freiburg (DE)
(74) Vertreter: Manitz, Gerhart, Dr.

(56) Entgegenhaltungen:
- EP-A- 0 495 140
- EP-A- 0 556 705
- JP-A- 7 124 101
- US-A- 2 979 581

## Beschreibung

Die Erfindung betrifft ein Verfahren zum Betrieb eines Hochfrequenz-Chirurgiegerätes nach dem Oberbegriff des Patentansprüches 1 sowie ein Hochfrequenz-Chirurgiegerät (vgl. JP-A-07124101).

Derartige Hochfrequenz-Chirurgiegeräte sollen universell für die verschiedensten Anwendungszwecke einsetzbar sein. Zu diesem Zweck weist ein Hochfrequenz-Chirurgiegerät Anschlüsse für ein oder mehrere monopolare, bipolare und/oder tripolare Hochfrequenz-Chirurgieinstrumente, sowie für den Anschluß einer Neutralelektrode auf. Unter tripolaren Instrumenten sind dabei solche zu verstehen, bei denen sich die Neutralelektrode am Instrument selbst befindet und mit denen wahlweise eine Koagulation oder ein Schneidvorgang durchgeführt werden kann, indem das Instrument dreipolig an den Hochfrequenzgenerator angeschlossen wird, um mittels zweier Koagulationselektroden oder einer Neutralelektrode und einer Schneidelektrode des Instrumentes wahlweise eine bipolare Koagulation oder ein bipolares Schneiden vornehmen zu können.

Außerdem ist an das Gerät im allgemeinen noch ein Fußschalter angeschlossen, mit welchem das betriebene Instrument wahlweise ein- oder ausgeschaltet werden kann.

Die interne Schaltung derartiger Hochfrequenz-Chirurgiegeräte ist dabei im allgemeinen so, daß beim gleichzeitigen Anschluß mehrerer Instrumente jeweils nur eines zu einer Zeit betrieben werden kann, während die anderen automatisch abgeschaltet werden. Dasjenige Instrument, welches zuerst eingeschaltet wird, hat dabei den Vorrang.

Für die unterschiedlichen Anwendungen müssen bei einem derartigen Hochfrequenz-Chirurgiegerät zahlreiche Schalt- und Anzeigemöglichkeiten vorhanden sein, damit der Operateur je nach dem verwendeten Instrument die gewünschte Betriebsart, z.B. monopolar, tripolar oder bipolar, die Funktion, wie Schneiden oder Koagulieren, und schließlich die gewünschte Charakteristik des Hochfrequenz-Strom-Spannungsverlaufes einstellen kann. Aufgrund dieser vielfältigen Schalt- und Anzeigemöglichkeiten sind die Bedienungsfelder derartiger Hochfrequenz-Chirurgiegeräte häufig sehr unübersichtlich, so daß die Bedienung hohe Aufmerksamkeit erfordert und es durch Fehlbedienung zu Problemen kommen kann.

Das Ziel der vorliegenden Erfindung besteht darin, ein Verfahren und ein Hochfrequenz-Chirurgiegerät der eingangs genannten Gattung zu schaffen, welches ein sehr übersichtliches Betätigungs- und Anzeigefeld aufweist, gleichwohl aber eine große Vielzahl unterschiedlicher Schalt- und Anzeigemöglichkeiten zuläßt, wie dies für einen universellen Einsatz des Gerätes erforderlich ist.

Zur Lösung dieser Aufgabe sind die Merkmale der Ansprüche 1 und 2 vorgesehen.

Vorteilhafte Ausführungsformen sind in den unteransprüchen beschrieben.

Besonders vorteilhaft ist die Erfindung anwendbar, wenn außer der Vielzahl von Betriebsarten und Charakteristiken auch noch eine Mehrzahl von Funktionen vorgegeben ist, wobei jeder Funktion ein eigener Satz Schalttasten zugeordnet ist. Die Funktionen werden zweckmäßigerweise durch Schalten am Instrument selbst ausgewählt.

Die Betriebsartenwahltasten können als Folgeschalttaste ausgeführt sein. Unter einer Folgeschalttaste ist dabei zu verstehen, daß zahlreiche Einzeltasten in einer Folgeschalttaste zusammengefaßt sind, wobei die dem Drücken einer Einzeltaste entsprechende Schaltung bei einer Folgeschalttaste durch mehrfaches Drücken der Folgeschalttaste nacheinander angewählt wird. Über der Folgeschalttaste vorgesehene Anzeigelämpchen, deren Zahl der Zahl möglicher Schaltvorgänge entspricht, können dabei vorzugsweise anzeigen, in welchem Schaltzustand sich der durch die Folgeschalttaste beaufschlagte Schalter befindet.

Wenn jeder Betriebsartenwahlschalter jeweils einem bestimmten Behandlungsinstrument zugeordnet ist, können bei Anwählen einer bestimmten Betriebsart auch weitere, das angewählte Behandlungsinstrument betreffende Funktionen ausgelöst werden können, wie z.B. eine Betriebsbereitschaftsanzeige am zugeordneten Instrument. Da bei Hochfrequenz-Chirurgiegeräten das Einschalten eines Instrumentes häufig durch einen äußeren Schalter, insbesondere einen Fußschalter vorgenommen wird, kann weiter vorgesehen sein, daß durch Anwählen einer bestimmten Betriebsart gleichzeitig der äußere Schalter dem durch die Betriebsartenwahltaste angewählten Instrument zugeordnet wird, so daß seitens des Chirurgen keinerlei Manipulationen vorgenommen werden müssen, um den äußeren Schalter richtig anzuschließen.

Hochfrequenz-Chirurgiegeräte umfassen im allgemeinen die monopolare Betriebsart, bei welcher außer dem eigentlichen Behandlungsinstrument auch eine Neutralelektrode angeschlossen ist, die an geeigneter Stelle mit dem Körper des Patienten in Berührung gebracht wird. Die Neutralelektrode ist innerhalb des Gerätes normalerweise mit einer Alarmvorrichtung verbunden, welche anspricht, wenn die Neutralelektrode nicht oder nicht einwandfrei mit dem Körper des Patienten in elektrischem Kontakt steht. Wird ein derartiges Hochfrequenz-Chirurgiegerät in einer anderen als der monopolaren Betriebsart verwendet, wird die Neutralelektrode nicht benötigt und befindet sich daher nicht in Kontakt mit dem Patienten. Da sie dennoch häufig an das Gerät angeschlossen bleibt, besteht das Problem, daß die Alarmvorrichtung anspricht und eine nicht einwandfreie Funktion des Gerätes vortäuscht.

Aus diesem Grunde sieht die Erfindung bei einer weiteren Ausführungsform vor, daß diese Alarmvorrichtung durch geeigneten Anschluß an die von den Betriebsartenwahltasten beaufschlagte Elektronik jeweils dann automatisch unwirksam gemacht wird, wenn durch Anwählen einer anderen Betriebsart als der monopolaren die monopolare Betriebsart verlassen wird.

Die Erfindung wird im folgenden beispielsweise anhand der Zeichnung beschrieben; in dieser zeigt:
- Figur 1: eine schematische Darstellung eines erfindungsgemäßen Hochfrequenz-Chirurgiegerätes und
- Figur 2: eine praktische Ausführungsform eines erfindungsgemäßen Hochfrequenz-Chirurgiegerätes in ebenfalls schematischer, blockschaltbildartiger Darstellung.

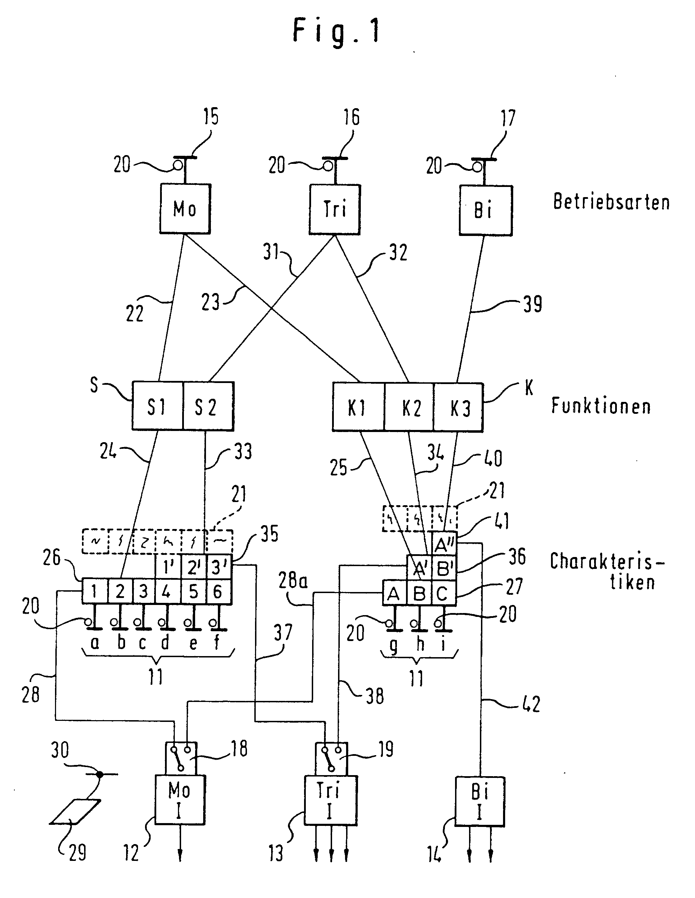

Nach Figur 1 weist ein erfindungsgemäßes Hochfrequenz-Chirurgiegerät drei vom auf der Vorderseite vorgesehenen Bedienungsfeld aus zugängliche Betriebsartenwahltasten 15, 16, 17 für die Betriebsarten monopolar, tripolar bzw. bipolar auf.

Die bei Betätigen der Betriebsartenwahltaste 15 gebildeten Signale sprechen über Pfade 22, 23 jeweils einen Funktionsblock S1 bzw. K1 an, welche für die Funktionen Scheiden bzw. Koagulieren stehen.

Von den Funktionsblöcken S1 und K1 führen Signalpfade 24, 25 zu Modifikationsblöcken 26 bzw. 27, welche von sechs bzw. drei Schalttasten 11a, b, c, d, e, f bzw. 11g, h, i ausgewählt werden können, die von der Frontseite des Gerätes her zugänglich sind.

Von den Modifikationsblöcken 26, 27 führen Signalpfade 28a bzw. 28 zu einem an einem Monopolar-Behandlungsinstrument 12 angeordneten Umschalter 18, welcher durch eine Bedienungsperson betätigbar ist und es gestattet, wahlweise den Signalpfad 28 oder den Signalpfad 28a an das Behandlungsinstrument 12 anzulegen. Die zum Monopolar-Behandlungsinstrument 12 gehörende Neutralelektrode 29 ist bei 30 in geeigneter Weise an das Gerät angeschlossen.

In entsprechender Weise führt von der Betriebsartenwahltaste 16 ein Signalpfad 31 zu einem Funktionsblock S2 für Schneiden und ein Signal 32 zu einem Funktionsblock K2 für Koagulieren. Von diesen Funktionsblöcken S2, K2 verlaufen Signalpfade 33 bzw. 34 zu Modifikationsblöcken 35 bzw. 36, die von den Schalttasten 11d, e, f bzw. 11h, i angesteuert werden. Von den Modifikationsblöcken 35, 36 erstrecken sich Signalpfade 37, 38 zu einem an einem Tripolar-Behandlungsinstrument 13 vorgesehenen Umschalter 19, welcher von der Bedienungsperson betätigt werden kann und das Instrument wahlweise an einen der Modifikationsblöcke 35 oder 36 anzuschließen gestattet.

Schließlich ist die Betriebsartenwahltaste 17 über einen Signalpfad 39 an einen Funktionsblock K3 für Koagulieren angeschlossen, von dem aus sich ein weiterer Signalpfad 40 zu einem einzigen Modifikationsblock 41 erstreckt, der von der Schalttaste 11i angesteuert wird. Vom Modifikationsblock 41 führt ein Signalpfad 42 zu einem an das Gerät angeschlossenen Bipolar-Behandlungsinstrument 14.

Jeder Schalttaste 15, 16, 17 und 11a bis i ist jeweils ein Anzeigelämpchen 20 zugeordnet, welches aufleuchtet, wenn die betreffende Schalttaste gedrückt und nicht in der sich aus im folgenden ergebenden Weise blockiert ist.

Jeder der Schalttasten 11a bis 11i ist außerdem ein Leuchtfeld 21 zugeordnet, welches aufleuchtet, wenn die betreffende Schalttaste anwählbar und nicht blockiert ist, und zwar unabhängig davon, ob die Schalttaste betätigt ist oder nicht.

Die Funktionsblöcke S1, S2 bilden zusammen eine Funktionsblockanordnung F, die Funktionsblöcke K1, K2 und K3 eine Funktionsblockanordnung K.

Die Modifikationsblöcke 26, 35, 27, 36, 41 weisen jeweils elektronische Stufen 1, 2, 3, 4, 5, 6; 1', 2', 3'; A, B, C; A', B' bzw. A'' auf, welche die für das jeweils angeschlossene Instrument bestimmte Hochfrequenz in der Weise modifizieren, daß eine für das betreffende Instrument optimal geeignete Abstufung von Hochfrequenzcharakteristiken, d.h. impedanzabhängigen Hochfrequenz-Strom-Spannungsverläufen erzielt wird.

Die Arbeitsweise des beschriebenen Hochfrequenz-Chirurgiegerätes ist wie folgt:

Wird die Betriebswahltaste 15 gedrückt, werden über die Funktionsblöcke S1 und K2 die Modifikationsblöcke 26 und 27 aktiviert, was bedeutet, daß die Schalttasten 11a bis 11f die elektronischen Stufen 1, 2, 3, 4, 5, 6 und die Schalttasten 11g, h, i die elektronischen Stufen A, B bzw. C innerhalb des nicht dargestellten Hochfrequenzgenerators anwählen können. Erfindungsgemäß kann von den Schalttasten 11a bis f bzw. 11b bis i jeweils nur eine betätigt werden. Nach einer solchen Betätigung sind die übrigen Schalttasten der beiden Sätze von Schalttasten blockiert.

Während die Funktionsblöcke S1 und K1 die elektronischen Anforderungen an die Funktionen Schneiden bzw. Koagulieren berücksichtigen und realisieren, stellen die elektronischen Stufen 1 bis 6 bzw. A bis C der Modifikationsblöcke 26, 27 die für den gewünschten Instrumentenbetrieb erforderliche Charakteristik des impedanzabhängigen Hochfrequenz-Strom-Spannungsverlaufs zur Verfügung. Geändert wird dabei in erster Linie die funktionelle Abhängigkeit der Leistung von der Impedanz und/oder die Modulation der verwendeten Hochfrequenz. Die elektronischen Funktionsblöcke S1 bzw. K1 sowie die elektronischen Stufen 1 bis 6 und A bis C sind dabei ganz auf die Bedürfnisse des monopolaren Behandlungsinstruments 12 abgestimmt und modifizieren die vom nicht-dargestellten Hochfrequenzgenerator erzeugten Parameter in vorbestimmter Weise.

Nach Drücken der Betriebswahltaste 15 sind die übrigen Betriebswahltasten 16, 17 blockiert.

Soll nun das Tripolar-Behandlungsinstrument 13 betrieben werden, wird lediglich die Betriebswahltaste 16 gedrückt, wodurch zunächst die vorher eingeschaltete Betriebsart deaktiviert und die elektronischen Funktionsblöcke S2 bzw. K2 sowie die Modifikationsblöcke 35 bzw. 36 aktiviert werden, deren elektronische Stufen 1', 2', 3' bzw. A', B' von den Schalttasten 11d, e, f, bzw. 11h, i anwählbar sind. Erfindungsgemäß werden also die elektronischen Stufen 4, 5, 6 und B, C von den gleichen Schalttasten 11d, e, f bzw. 11h, i angewählt wie die vorgenannten elektronischen Stufen 1', 2', 3' bzw. A', B'. Für zwei unterschiedliche Modifikationsblöcke 26 bzw. 35 und 27 bzw. 36 wird also der gleiche Satz von Schalttasten 11 herangezogen.

Nachdem das tripolare Behandlungselement 13 lediglich drei verschiedene Abstufungen bei der Charakteristik des Hochfrequenz-Strom-Spannungsverlaufs im Funktionsmodus Schneiden und nur zwei Abstufungen im Funktionsmodus Koagulieren erfordert, sind erfindungsgemäß bei Drücken der Schalttaste 16 die Schalttasten 11a, b, c und 11g blockiert. Dies wird in den zugeordneten Leuchtfeldern 21 dadurch zur Anzeige gebracht, daß diese nicht aufleuchten, während die Leuchtfelder 21, die den elektronischen Stufen 1', 2', 3' bzw. A', B' zugeordnet sind, bei Aktivierung der betreffenden Schalttastenblöcke 35, 36 aufleuchten und vorzugsweise symbolisch die nunmehr durch gezieltes Drücken der Schalttasten möglichen Behandlsungsvorgänge darstellen.

Durch Umlegen der Schalter 18 bzw. 19 können wahlweise die Tastenfelder 11a bis f oder 11g bis i in der umfassenden Form (26, 27) oder in der eingeschränkten Form (35, 36) wirksam gemacht werden.

Die dritte Möglichkeit bei dem in Figur 1 dargestellten Ausführungsbeispiel besteht darin, daß die Bipolar-Betriebswahltaste 17 gedrückt wird, worauf über den Signalpfad 39, den elektronischen Funktionsblock K3 und den Signalpfad 40 ein weiterer Modifikationsblock 41 aktiviert wird, welcher in diesem Ausführungsbeispiel lediglich eine einzige elektronische Stufe A'' aufweist, die von der gleichen Schalttaste 11i anwählbar ist, wie die elektronischen Stufen B' und C'. Beim Anwählen beaufschlagt die elektronische Stufe A'' über den Signalpfad 42 das Bipolar-Behandlungsinstrument 14 und versorgt es mit einem geeigneten Hochfrequenz-Strom-Spannungsverlauf.

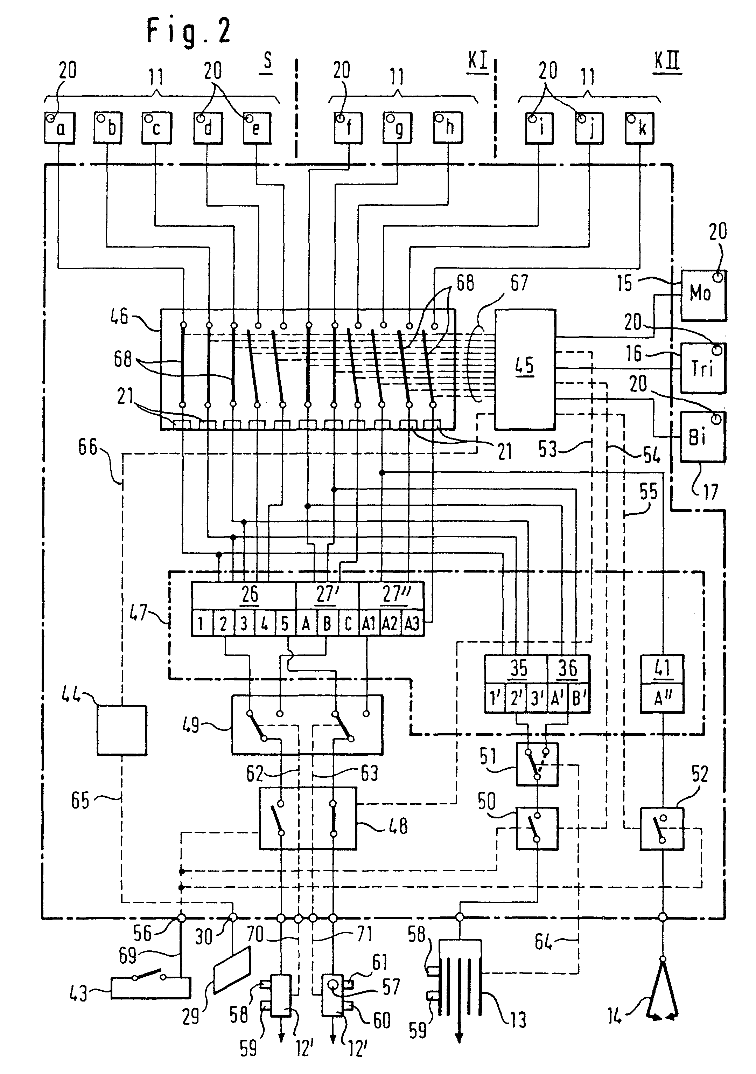

In der Ausführungsform nach Figur 2 bezeichnen gleiche Bezugszahlen entsprechende Elemente wie in Figur 1.

Nach Figur 2 sind die Betriebswahltasten 15, 16, 17 an eine Elektronik 45 angeschlossen, welche über einen Steuerleitungssatz 67 eine Tasten-Blockier-/Deblockierstufe 46 beaufschlagt, die für jede Schalttaste 11a bis 11k einen individuell angesteuerten Ein-Aus-Schalter 68 enthält. Die Schalttasten 11a bis 11k sind an die einzelnen Schalter 68 der Tasten-Blockier-/Deblockierstufe 46 angeschlossen, so daß in Abhängigkeit von der gedrückten Betriebsartenwahltaste 15, 16 oder 17 und der dadurch individuell geöffneten oder geschlossenen Schalter 68 eine vorbestimmte Anzahl von Schalttasten 11 durchgeschaltet oder blockiert wird. Die Schalttasten 11a bis 11e sind der Funktion S "Schneiden", die Schalttasten 11f bis 11h einer Funktion KI "Koagulieren" und die Schalttasten 11i bis 11k einer zweiten Funktion KII "Koagulieren" zugeordnet.

Bei der in Figur 2 wiedergegebenen Schaltposition der Stufe 46 ist angenommen, daß die Betriebsartenwahltaste 16 für das Tripolarinstrument 13 gedrückt ist, was zu einem Anwählen der Schalttasten 11a, b, c des Funktionsmodus S und der Schalttasten 11f, 11g des Funktionsmodus KI führt. Die übrigen Schalttasten 11d, e und 11h, i, j, k sind in diesem Fall durch Öffnen der in der Stufe 46 der entsprechenden Schalter 68 blockiert.

Im eigentlichen Hochfrequenz-Generator 47, der durch eine strichpunktierte Linie umgrenzt ist, sind unter anderem vorgesehen:
- ein Modifikationsblock 26 mit elektronischen Stufen 1, 2, 3, 4, 5;
- ein Modifikationsblock 27' mit elektronischen Stufen A, B, C;
- ein Modifikationsblock 27'' mit elektronischen Stufen A1, A2 und A3;
- ein Modifikationsblock 35 mit elektronischen Stufen 1', 2', 3';
- ein Modifikationsblock 36 mit elektronischen Stufen A', B' und
- ein Modifikationsblock 41 mit einer elektronischen Stufe A''.

Im Unterschied zum Prinzip-Blockschaltbild nach Figur 1 weist der Modifikationsblock 26 nach Figur 2 nur fünf unterschiedliche Charakteristiken liefernde elektronische Stufen 1 - 5 auf, und außerdem sind für den Funktionsmodus "Koagulieren" zwei jeweils den Schalttastensätzen 11f - 11h bzw. 11i - 11k zugeordnete unterschiedliche Modifikationsblöcke 27', 27'' mit jeweils unterschiedliche Charakteristiken aufweisenden elektronischen Stufen A, B, C bzw. A1, A2, A3 vorgesehen.

Im weiteren Unterschied zu Figur 1 sind an das Hochfrequenz-Chirurgiegerät nach Figur 2 zwei monopolare Behandlungsinstrumente 12', 12'' angeschlossen, welche über eine Umschaltvorrichtung 48 und einen Funktionswahlschalter 49 in der aus Figur 2 ersichtlichen Weise an die Modifikationsblöcke 26, 27', 27'' angeschlossen sind.

Das Tripolar-Behandlungsinstrument 13 ist über eine Schaltvorrichtung 50 und einen Funktionswahlschalter 51 an die Modifikationsblöcke 35, 36 angeschlossen. Schließlich ist ein an das Gerät angelegtes Bipolar-Behandlungsinstrument 14 über eine Schaltvorrichtung 52 mit dem Modifikationsblock 41 verbunden.

Von der Elektronik 45 führen in Figur 2 gestrichelt angedeutete Steuerleitungen 53, 54, 55 zu den Schaltvorrichtungen 48, 50 bzw. 52, welche bei Drücken der zugeordneten Betriebsartenwahltaste 15, 16 oder 17 das jeweilige Behandlungselement 12', 12'', 13 oder 14 mit dem Hochfrequenz-Generator 47 verbinden. Da an die Umschaltvorrichtung 48 zwei Behandlungsinstrumente 12', 12'' angeschlossen sind, ist die Schaltvorrichtung 48 außerdem so ausgebildet, daß von den beiden Monopolar-Behandlungsinstrumenten 12', 12'' jeweils nur eines angeschlossen wird, und zwar vorzugsweise das, welches von den behandelnden Chirurgen als erstes betätigt wird.

Weiter ist in Figur 2 der für das Ein- und Ausschalten des jeweils angeschlossenen Behandlungsinstrumentes 12', 13 oder 14 vorgesehene Fußschalter 43 schematisch dargestellt, welcher über ein Kabel 69 an eine Buchse 56 des Gerätes angeschlossen ist.

Der Fußschalter 43 ist wahlweise an eines der Behandlungsinstrumente 12' oder 12'', 13, 14 anlegbar, was durch die auf das Drücken der Betriebsartwahltasten 15, 16, 17 reagierende Elektronik 45 ebenfalls über die Steuerleitungen 53, 54, 55 veranlaßt wird. Auf diese Weise wird erreicht, daß der Fußschalter 43 jeweils automatisch an dasjenige Behandlungsinstrument angeschlossen ist, welches durch Anwahl der zugeordneten Betriebsartenwahltaste 15, 16 oder 17 gerade betrieben werden soll.

Bezüglich der Behandlungsinstrumente 12', 12'' kann vorher festgelegt werden, welches der beiden Instrumente durch den Fußschalter 43 und welches durch einen beispielsweise am Instrument 12'' vorhandenen Handschalter 57 betätigt werden soll.

Schließlich ist in Figur 2 noch durch gestrichelte Linien 62, 63 angedeutet, wie mittels an den Behandlungsinstrumenten 12', 12'' vorgesehenen Schaltköpfen 58, 59 bzw. 60, 61 der Funktionswahlschalter 49 in die Position "Schneiden" und "Koagulieren" umgesteuert werden kann. Die Schaltköpfe 58, 59 bzw. 60, 61 sind über sich zum Funktionswahlschalter 48 erstreckende Steuerleitungen 70, 71 wirksam.

Auch das Tripolar-Behandlungsinstrument 13 weist zwei derartige Schaltknöpfe 58, 59 auf, welche über eine Steuerleitung 64 den Funktionswahlschalter 51 in die Position "Schneiden" oder "Koagulieren" stellen können.

Weiter ist in Figur 2 noch die bei 30 angeschlossene Neutralelektrode 29 für das monopolare Schneiden angedeutet, deren Beaufschlagung durch Hochfrequenz nicht im einzelnen dargestellt ist. Gezeigt ist jedoch eine gestrichelt angedeutete Steuerleitung 65, welche zu einer Alarmvorrichtung 44 führt, die außerdem über eine weitere Steuerleitung 66 von der Elektronik 45 beaufschlagt wird.

Die Alarmvorrichtung 44 spricht in der monopolaren Betriebsart dann an, wenn die Neutralelektrode 29 nicht oder nicht einwandfrei in elektrischem Kontakt mit dem Körper des Patienten steht. Da dies bei den Betriebsarten tripolar und bipolar regelmäßig der Fall ist, sorgt die Elektronik 45 dafür, daß die Alarmvorrichtung 44 dann deaktiviert wird, wenn die Betriebsartenwahltaste 15 nicht gedrückt ist. Auf diese Weise wird bei Drücken der Betriebsartenwahltasten 16, 17 und entsprechendem Deaktivieren der Betriebsartenwahltaste 15 die Alarmvorrichtung 44 abgeschaltet.

Wie erwähnt sind beim Ausführungsbeispiel nach Figur 2 zwei Schalttastensätze 11f, g, h bzw. 11i, j, k für zwei Koagulations-Funktionsmodi K1 bzw. K2 vorgesehen. Die Schalttasten 11f, g sind dabei im Falle der Aktivierung der Tripolar-Betriebsart an das Tripolar-Instrument 13 angeschlossen, während von dem Schalttastensatz 11i, j, k bei Drücken der Bipolar-Betriebsartenwahltaste 17 nur die Schalttaste 11i an den Modifikationsblock 41 für das Bipolar-Behandlungselement 14 angeschlossen ist. Grundsätzlich könnte hierfür auch eine der Schalttasten 11f, g, h verwendet werden.

Erfindungsgemäß hat das Vorsehen von zwei Sätzen von Schalttasten für den Funktionsmodus KI "Koagulation" und für den Funktionsmodus KII "Koagulation" den Sinn, daß bei gleichzeitigem Anschluß von zwei Monopolar-Behandlungsinstrumenten 12', 12'' jeweils ein individueller Modifikationsblock 27' bzw. 27'' für jedes Instrument 12', 12'' zur Verfügung steht. Auf diese Weise werden die Behandlungsinstrumente 12', 12'' zwar im Modus "Schneiden" vom gleichen Modifikationsblock 26 beeinflußt, so daß die Schneidcharakteristiken beider Instrumente gleich sind. Für die Koagulation können jedoch in den Elektrodenstufen A, B, C bzw. A1, A2, A3 unterschiedliche Charakteristiken für die beiden Behandlungsinstrumente 12', 12'' im Koagulationsmodus vorgesehen werden. Dies stellt einen wesentlichen Vorteil dar, weil hierdurch bei einer bestimmten Operation zwei Behandlungsinstrumente 12', 12'' zur Verfügung stehen, mit denen unterschiedlichen Anforderungen an ihre Charakteristik bei der Koagulation entsprochen werden kann.

Zu betonen ist, daß für den Fall, daß sämtliche Instrumente in der aus Figur 2 ersichtlichen Weise angeschlossen sind, zu einer Zeit jeweils immer nur eines der Instrumente in Betrieb sein kann. Dies wird durch Drücken der entsprechenden Betriebsartenwahltaste herbeigeführt, wobei durch Drücken der Betriebsartenwahltaste 15 zwar beide Behandlungsinstrumente 12', 12'' angewählt werden, jedoch geräteintern vorgesehen ist, daß immer nur das zuerst eingeschaltete Instrument arbeitet, während das andere dann automatisch im abgeschalteten Zustand gehalten wird.

Aufgrund der erweiterten Anordnung nach Figur 2 ist es möglich, daß die Betriebsartenwahltasten 15, 17 auch gleichzeitig angewählt werden können, denn in diesem Fall werden die Schalttasten 11a, b, c, d, e; 11f, g, h für das monopolare Behandlungsinstrument 12' oder 12'' und 11i für das bipolare Behandlungsinstrument 14 deblockiert, so daß durch Anschließen von Behandlungsinstrumenten 12', 12'' und 14 drei Instrumente für den Gebrauch bereit stehen, ohne daß es der Betätigung einer weiteren Betriebsartenwahltaste bedarf.

Aufgrund derselben Überlegungen könnten auch die Betriebsartenwahltasten 16, 17 gleichzeitig gedrückt werden, weil dann ebenfalls nur voneinander entkoppelte Schalttasten 11 angewählt werden.

Im übrigen ist die Arbeitsweise der Ausführungsform nach Figur 2 gleich der anhand von Figur 1 beschriebenen.

### Bezugszeichenliste

- 11a: Schalttaste
- 11b: Schalttaste
- 11c: Schalttaste
- 11d: Schalttaste
- 11e: Schalttaste
- 11f: Schalttaste
- 11g: Schalttaste
- 11h: Schalttaste
- 11i: Schalttaste
- 11j: Schalttaste
- 11k: Schalttaste
- 12: monopolares Behandlungsinstrument
- 12': monopolares Behandlungsinstrument
- 12'': monopolares Behandlungsinstrument
- 13: tripolares Behandlungsinstrument
- 14: bipolares Behandlungsinstrument
- 15: Betriebsartwahlschalter
- 16: Betriebsartwahlschalter
- 17: Betriebsartwahlschalter
- 18: Umschalter
- 19: Umschalter
- 20: Anzeigelämpchen
- 21: Leuchtfeld
- 22: Signalpfad
- 25: Signalpfad
- 26: Modifikationsblock
- 27: Signalpfad
- 28: Signalpfad
- 29: Neutralelektrode
- 30: Anschluß
- 31: Signalpfad
- 32: Signalpfad
- 33: Signalpfad
- 34: Signalpfad
- 35: Modifikationsblock
- 36: Modifikationsblock
- 37: Signalpfad
- 38: Signalpfad
- 39: Signalpfad
- 40: Signalpfad
- 41: Modifikationsblock
- 42: Signalpfad
- 43: Fußschalter
- 44: Alarmvorrichtung
- 45: Elektronik
- 46: Tasten-Blockier-/Deblockierstufe
- 47: Hochfrequenz-Generator
- 48: Umschaltvorrichtung
- 49: Funktionswahlschalter
- 50: Schaltvorrichtung
- 51: Funktionswahlschalter
- 52: Schaltvorrichtung
- 53: Steuerleitung
- 54: Steuerleitung
- 55: Steuerleitung
- 56: Buchse
- 57: Schalter
- 58: Schaltknopf
- 59: Schaltknopf
- 60: Schaltknopf
- 61: Schaltknopf
- 62: Steuerleitung
- 63: Steuerleitung
- 64: Steuerleitung
- 65: Steuerleitung
- 66: Steuerleitung
- 67: Steuerleitungssatz
- 68: Schalter
- 69: Kabel
- 70: Steuerleitung
- 71: Steuerleitung

## Patentansprüche

1. Verfahren zum Betrieb eines Hochfrequenz-Chirurgiegerätes mit mehreren auswählbaren Betriebsarten (Mo, Tri, Bi), wie monopolar, tripolar, bipolar, und einer Vielzahl von Schalttasten (11), welche für jede Betriebsart (Mo, Tri, Bi) das Auswählen einer bestimmten Anzahl von individuellen Charakteristiken gestatten, in jeder von denen ein der betreffenden Betriebsart (Mo, Tri, Bi) entsprechendes Hochfrequenz-Behandlungs-Instrument (12, 13, 14) mit einem der individuellen Charakteristik entsprechenden gewünschten impedanzabhängigen Hochfrequenz-Spannungs-Stromverlauf gespeist wird, wobei
zumindest eine und vorzugsweise mehrere Schalttasten (11) mehreren Betriebsarten (Mo, Tri, Bi) zugeordnet werden und in jeder Betriebsart ein vorbestimmter Teil der Schalttasten (11) anwählbar und der Rest der Schalttasten blockiert wird
**dadurch gekennzeichnet, daß**
jeder Schalttaste (11) eine Anzeigevorrichtung (21) zugeordnet wird, welche anzeigt, ob die zugeordnete Schalttaste (11) in der ausgewählten Betriebsart (Mo, Tri, Bi) anwählbar oder blockiert ist, und daß jeder Schalttaste (11) jeweils eine Anzeigevorrichtung (20) zugeordnet wird, welche anspricht, wenn die betreffende Schalttaste (11) gedrückt und nicht blockiert ist.

2. Hochfrequenz-Chirurgiegerät mit mehreren unterschiedlichen Betriebsarten (Mo, Tri, Bi), wie monopolar, tripolar, bipolar, wenigstens einer Funktion (S, K), wie Schneiden, Koagulieren, in jeder Betriebsart (Mo, Tri, Bi) und wenigstens einer Betriebsart (Mo, Tri) mit mehreren Funktionen (S1, K1; S2, K2) sowie wenigstens einer durch jeweils eine Schalttaste (11) anwählbaren Charakteristik (1 - 6, 1' - 3'; A - C; A', B'; A") innerhalb jeder Funktion (S1, S2; K1, K2, K3), wobei für jede Betriebsart (Mo, Tri, Bi) eine Betriebsartwahltaste (15, 16, 17) vorgesehen ist, zumindest ein Teil der Schalttasten (11) mehreren Betriebsartenwahltasten (15, 16, 17) zugeordnet ist und bei Betätigung wenigstens einer der Betriebsartenwahltasten (15, 16, 17) wenigstens eine und vorzugsweise mehrere der Schalttasten (11) blockiert sind, **dadurch gekennzeichnet, daß** jeder Schalttaste (11) eine Anzeigevorrichtung (21) zugeordnet ist, welche anzeigt, ob die zugeordnete Schalttaste (11) in der ausgewählten Betriebsart (Mo, Tri, Bi) anwählbar oder blockiert ist, und wobei jeder Schalttaste (11) jeweils eine Anzeigevorrichtung (20) zugeordnet ist, welche anspricht, wenn die betreffende Schalttaste (11) gedrückt und nicht blockiert ist.

3. Hochfrequenz-Chirurgiegerät nach Anspruch 2,
**dadurch gekennzeichnet,daß**
bei Betätigung einer der zu einer Funktion (S, K) gehörenden Schalttasten (11) und/oder einer der Betriebsartenwahltasten (15, 16, 17) die übrigen, der gleichen Funktion (S, K) zugeordneten Schalttasten bzw. die übrigen Betriebsartenwahltasten blockiert sind.

4. Hochfrequenz-Chirurgiegerät nach Anspruch 2 oder 3,
**dadurch gekennzeichnet, daß**
die Schalttasten (11) jeweils eine die im Zusammenhang mit der angewählten Betriebsart und Funktion erwünschte Charakteristik (1 - 6; 1' - 3'; A - C; A', B'; A") ergebende elektronische Schaltstufe individuell ansteuern.

5. Hochfrequenz-Chirurgiegerät nach einem der Ansprüche 2 bis 4,
**dadurch gekennzeichnet,daß**
die Betriebsartenwahltasten (15, 16, 17) sowie die Schalttasten (11) über einen Datenbus mit einem Mikro-Controller verbunden sind, der die durch Tastendruck ausgewählten Charakteristiken (1 - 6; 1' - 3'; A - C; A', B'; A") anwählt und für eine entsprechende Speisung eines angeschlossenen Instrumentes (12, 12', 12", 13, 14) sorgt.

6. Hochfrequenz-Chirurgiegerät nach einem der Ansprüche 2 bis 5,
**dadurch gekennzeichnet,daß**
jeder Betriebsartenwahltaste (15, 16, 17) und jeder Schalttaste (11) eine Anzeigevorrichtung (20) insbesondere Anzeigelampe zugeordnet ist, welche anspricht, wenn die betreffende Betriebsartenwahltaste (15, 16, 17) und/oder Schalttaste (11) gedrückt und die betreffende Betriebsart (Mo, Tri, Bi) bzw. eine Charakteristik (1 - 6; 1' - 3'; A - C; A', B'; A") angewählt ist.

7. Hochfrequenz-Chirurgiegerät nach einem der Ansprüche 2 bis 6,
**dadurch gekennzeichnet,daß**
die jeder Schalttaste (11) zugeordnete Anzeigevorrichtung ein Leuchtfeld (21) ist.

8. Hochfrequenz-Chirurgiegerät nach einem der Ansprüche 2 bis 7,
**dadurch gekennzeichnet, daß**
ein an das Gerät angeschlossener äußerer Schalter (43), insbesondere Fußschalter, für das Ein- und Ausschalten angeschlossener Behandlungsinstrumente (12, 12', 13, 14) durch das Betätigen einer der Betriebsartenwahltasten (15, 16, 17) dem dadurch angewählten Behandlungsinstrument (12, 12', 13 bzw. 14) zuordenbar ist.

9. Hochfrequenz-Chirurgiegerät nach einem der Ansprüche 2 bis 8,
**dadurch gekennzeichnet,daß**
eine monopolare Betriebsart und eine Alarmvorrichtung (44) vorgesehen ist, welche anspricht, wenn eine Neutralelektrode (29) sich nicht in einwandfreiem elektrischen Kontakt mit dem Patienten befindet, wobei die Alarmvorrichtung (44) bei Deaktivierung der monopolaren Betriebsart unwirksam gemacht wird.

## Claims

1. Method of operating a high-frequency surgical apparatus having a plurality of operating modes (Mo, Tri, Bi), such as monopolar, tripolar, bipolar, and a plurality of switch keys (11) which permit, for each operating mode (Mo, Tri, Bi), the selection of a specific number of individual characteristics mode, in each of which a desired, impedance dependent, high-frequency voltage/current characteristic corresponding to the individual characteristics is fed to a high-frequency treatment instrument (12, 13, 14) corresponding to the relevant operating mode (Mo, Tri, Bi),
wherein at least one and preferably a plurality of switching keys (11) are associated with a plurality of operating modes (Mo, Tri, Bi) and a predetermined number of the switching keys (11) can be selected in each operating mode and the rest of the switching keys are blocked,
**characterized in that**
a display device (21) is associated with each switching key (11) and indicates whether the associated switching key (11) can be selected in the selected operating mode (Mo, Tri, Bi) or is blocked; and **in that** an indicator device (20) is associated with each switching key (11) and responds when the relevant switching key (11) is pressed and is not blocked.

2. High-frequency surgical apparatus having a plurality of different operating modes (Mo, Tri, Bi), such as monopolar, tripolar, bipolar, at least one function (S, K) such as cutting or coagulating in each operating mode (Mo, Tri, Bi), at least one operating mode (Mo, Tri,) with a plurality of functions (S1, K1; S2, K2) and also at least one characteristic (1-6, 1'-3'; A-C; A', B'; A") within each function (S1, S2; K1, K2, K3) which can in each case be selected by a switching key (11), wherein an operating mode selection key (15, 16, 17) is provided for each operating mode (Mo, Tri, Bi), at least some of the switching keys (11) are associated with a plurality of operating mode selection keys (15, 16, 17), and on actuating at least one of the operating mode selection keys (15, 16, 17) at least one and preferably a plurality of the switching keys (11) are blocked, **characterized in that**
a display device (21) is associated with each switching key (11) and indicates whether the associated switching key (11) can be selected in the selected operating mode (Mo, Tri, Bi) or is blocked; and **in that** an indicator device (20) is associated with each switching key (11) and responds when the relevant switching key (11) is pressed and is not blocked.

3. High-frequency surgical apparatus in accordance with claim 2,
**characterized in that** on actuating one of the switching keys (11) belonging to a function (S, K) and/or one of the operating mode selection keys (15, 16, 17) the remaining switching keys associated with the same function (S, K) and/or the remaining operating mode selection keys are blocked.

4. High-frequency surgical apparatus in accordance with claim 2 or claim 3, **characterized in that** the switching keys (11) each individually control an electronic switching stage which results in the characteristic (1-6; 1'-3'; A-C; A', B'; A") desired in connection with the selected operating mode and function.

5. High-frequency surgical apparatus in accordance with any one of claims 2 to 4, **characterized in that** the operating mode selection keys (15, 16, 17) and also the switching keys (11) are connected via a databus to a microcontroller which selects the characteristics (1-6; 1'-3'; A-C; A', B'; A") selected by key pressure and takes.care of an appropriate feeding of the connected on instrument (12, 12', 12", 13, 14).

6. High-frequency surgical apparatus in accordance with any one of claims 2 to 5, **characterized in that** an indicator device (20), in particular an indicator lamp, is associated with each operating mode selection key (15, 16, 17) and each switching key (11) and responds when the relevant operating mode selection key (15, 16, 17) and/or switching key (11) is pressed and the relevant operating mode (Mo, Tri, Bi) or a characteristic (1-6; 1'-3'; A-C; A', B'; A") is selected.

7. High-frequency surgical apparatus in accordance with any one of claims 2 to 6, **characterized in that** the display device associated with each switching key (11) is a luminous field (21).

8. High-frequency surgical apparatus in accordance with any one of claims 2 to 7, **characterized in that** an external switch (43) connected to the instrument, in particular a foot switch, for the switching on or switching off of the attached treatment instruments (12, 12', 13, 14) is associated by the actuation of one of the operating mode selection keys (15, 16, 17) with the thereby selected or dialed in treatment instrument (12, 12', 13 and 14 respectively).

9. High-frequency surgical apparatus in accordance with any of claims 2 to 8, in which the monopolar operating mode and an alarm device (44) is provided, which responds when the neutral electrode (29) is not in a trouble-free electrical contact with the patient, **characterized in that** the alarm device (44) is made inactive on deactivating the monopolar operating mode.

## Revendications

1. Procédé de mise en oeuvre d'un appareil chirurgical à haute fréquence avec plusieurs modes de fonctionnement sélectionnables (Mo, Tri, Bi), comme monopolaire, tripolaire, bipolaire, et avec plusieurs touches de commutation (11) qui permettent pour chaque mode de fonctionnement (Mo, Tri, Bi) la sélection d'un certain nombre de caractéristiques individuelles, dans lequel dans chacun des modes de fonctionnement un instrument de travail à haute fréquence (12, 13, 14) correspondant au mode de fonctionnement concerné (Mo, Tri, Bi) est alimenté avec une allure de courant-tension haute fréquence souhaitée, dépendante de l'impédance et correspondant à la caractéristique individuelle,
dans lequel au moins une et de préférence plusieurs touches de commutation (11) sont associées à plusieurs modes de fonctionnement (Mo, Tri, Bi) et, dans chaque mode de fonctionnement, une partie prédéterminée des touches de commutation (11) est sélectionnable et le reste des touches de commutation est bloqué,
**caractérisé en ce qu'**il est associé à chaque touche de commutation (11) un dispositif d'affichage (21) qui indique si la touche de commutation associée (11) est sélectionnable ou bloquée dans le mode de fonctionnement sélectionné (Mo, Tri, Bi) et **en ce qu'**il est associé à chaque touche de commutation (11) un dispositif d'affichage (20) qui réagit lorsque la touche de commutation concernée (11) est actionnée et non bloquée.

2. Appareil chirurgical à haute fréquence avec plusieurs modes de fonctionnement différents (Mo, Tri, Bi), comme monopolaire, tripolaire, bipolaire, avec au moins une fonction (S, K), comme couper, coaguler, dans chaque mode de fonctionnement (Mo, Tri, Bi) et avec au moins un mode de fonctionnement (Mo, Tri) comportant plusieurs fonctions (SI, K1 ; S2, K2) ainsi qu'au moins une caractéristique (1 à 6, 1' à 3' ; A à C ; A', B' ; A") sélectionnable à chaque fois par une touche de commutation (11) à l'intérieur de chaque fonction (SI, S2 ; K1, K2, K3), dans lequel il est prévu pour chaque mode de fonctionnement (Mo, Tri, Bi) une touche de sélection de mode de fonctionnement (15, 16, 17),
dans lequel au moins une partie des touches de commutation (11) est associée à plusieurs touches de sélection de mode de fonctionnement (15, 16, 17) et dans lequel, lors de l'actionnement d'au moins l'une des touches de sélection de mode de fonctionnement (15, 16, 17), au moins une et de préférence plusieurs des touches de commutation (11) sont bloquées,
**caractérisé en ce qu'**il est associé à chaque touche de commutation (11) un dispositif d'affichage (21) qui indique si la touche de commutation associée (11) est sélectionnable ou bloquée dans le mode de fonctionnement sélectionné (Mo, Tri, Bi) et **en ce qu'**il est associé à chaque touche de commutation (11) un dispositif d'affichage (20) qui réagit lorsque la touche de commutation concernée (11) est actionnée et non bloquée.

3. Appareil chirurgical à haute fréquence selon la revendication 2,
**caractérisé en ce que**, lors de l'actionnement de l'une des touches de commutation (11) associée à une fonction (S, K) et/ou de l'une des touches de sélection de mode de fonctionnement (15, 16, 17), les autres touches de commutation associées à la même fonction (S, K) ou les autres touches de sélection de mode de fonctionnement sont bloquées.

4. Appareil chirurgical à haute fréquence selon la revendication 2 ou 3,
**caractérisé en ce que** les touches de commutation (11) commandent individuellement chacune un niveau de commutation électronique donnant la caractéristique (1 à 6 ; 1' à 3' ; A à C ; A', B' ; A") souhaitée en relation avec le mode de fonctionnement et la fonction sélectionnés.

5. Appareil chirurgical à haute fréquence selon l'une des revendications 2 à 4,
**caractérisé en ce que** les touches de sélection de mode de fonctionnement (15, 16, 17) ainsi que les touches de commutation (11) sont reliées par l'intermédiaire d'un bus de données à un microcontrôleur qui sélectionne les caractéristiques (1 à 6 ; 1' à 3' ; A à C ; A', B' ; A") sélectionnées par l'actionnement de la touche et qui se charge d'assurer une alimentation appropriée d'un instrument raccordé (12, 12', 12", 13, 14).

6. Appareil chirurgical à haute fréquence selon l'une des revendications 2 à 5,
**caractérisé en ce qu'**il est associé à chaque touche de sélection de mode de fonctionnement (15, 16, 17) et à chaque touche de commutation (11) un dispositif d'affichage (20), notamment une lampe témoin, qui réagit lorsque la touche de sélection de mode de fonctionnement concernée (15, 16, 17) et/ou la touche de commutation concernée (11) est actionnée et lorsque le mode de fonctionnement concerné (Mo, Tri, Bi) et/ou une caractéristique (1 à 6 ; 1' à 3'; A à C ; A', B' ; A") est sélectionné.

7. Appareil chirurgical à haute fréquence selon l'une des revendications 2 à 6,
**caractérisé en ce que** le dispositif d'affichage associé à chaque touche de commutation (11) est un champ lumineux (21).

8. Appareil chirurgical à haute fréquence selon l'une des revendications 2 à 7,
**caractérisé en ce qu'**un interrupteur externe (43) raccordé à l'appareil, notamment un interrupteur à commande au pied, pour la mise en marche et l'arrêt d'instruments de travail raccordés (12, 12', 13, 14) peut être associé, par l'actionnement de l'une des touches de sélection de mode de fonctionnement (15, 16, 17), à l'instrument de travail (12, 12', 13 ou 14) alors sélectionné.

9. Appareil chirurgical à haute fréquence selon l'une des revendications 2 à 8,
**caractérisé en ce qu'**il est prévu un mode de fonctionnement monopolaire et un dispositif d'alerte (44) qui réagit lorsqu'une électrode neutre (29) ne se trouve pas en contact électrique parfait avec le patient, le dispositif d'alerte (44) étant rendu inactif lors de la désactivation du mode de fonctionnement monopolaire.
